# EUROPEAN PATENT APPLICATION

(11) **EP 4 421 814 A1**
(43) Date of publication of application: **28.08.2024**
(21) Application number: 22884045.0
(22) Date of filing: 20.10.2022
(51) Int. Cl.: G16C 20/70, G16C 20/90, C12Q 1/6844

(54) **DEVICE AND METHOD FOR READING POSITIVE/NEGATIVE WITH RESPECT TO TARGET ASSAY SUBSTANCE IN SAMPLE**

(30) Priority: 21.10.2021 KR 20210141223
(71) Applicant: Seegene, Inc., Seoul 05548 (KR)
(72) Inventor: PARK, Bong Gyun, Seoul 05548 (KR); SEO, Ilhwan, Seoul 05548 (KR); SON, Hyungsuk, Seoul 05548 (KR); KIM, Kwangmin, Seoul 05548 (KR); CHA, Kwang Su, Seoul 05548 (KR); KIM, Sang Won, Seoul 05548 (KR); LYU, Won Woo, Seoul 05548 (KR)
(74) Representative: Viering, Jentschura & Partner mbB Patent- und Rechtsanwälte
(86) International application number: PCT/KR2022/015987
(87) International publication number: WO 2023/068823

(57) **Abstract**

A positive/negative determining apparatus according to one embodiment of the present invention includes: an input unit that receives signal values from a signal-generating reaction of a plurality of cycles targeting a target analyte, wherein the signal-generating reaction generates a signal dependent on a presence of the target analyte in a sample; a positive/negative determining unit pre-trained to estimate presence/ absence of the target analyte in the sample from the signal values in the plurality of cycles and a sequence of the plurality of cycles.

## Description

### TECHNICAL FIELD

The present disclosure relates to a device and method for reading positive/negative with respect to a target assay substance in a sample.

### BACKGROUND ART

The most commonly used nucleic acid amplification reaction, known as polymerase chain reaction (PCR), involves repeated cycles of denaturation of double-stranded DNA, annealing of oligonucleotide primer to a DNA template, and primer extension by DNA polymerase (Mullis et al., US Patent Nos. 4,683,195, 4,683,202 and 4,800,159; Saiki et al., Science 230:1350-1354 (1985)).

Real-time PCR is a PCR-based technology for detecting a target nucleic acid in a sample in real time. In order to detect a specific target nucleic acid, a signal generation means that emits a detectable fluorescent signal in proportion to the amount of the target nucleic acid during a PCR reaction is used. A fluorescent signal in proportion to the amount of the target nucleic acid is detected at each measurement point (cycle) through real-time PCR, a data set including each measurement point and a signal value at the measurement point is obtained, and an amplification curve or an amplification profile curve indicating the intensity of the detected fluorescence signal compared to the measurement point is obtained from the data set.

In general, an amplification curve by real-time PCR is divided into a baseline region, an exponential region, and a plateau region. The exponential region is a region where an emitted fluorescence signal increases in proportion to an increase in PCR amplification products, and the plateau region is a region where the increase in the PCR amplification products and emission of the fluorescence signal reach saturation and thus no further increase in the fluorescence signal appears. The baseline region refers to a region where the fluorescence signal remains constant without change at the beginning of the reaction. Since the baseline region is not sufficient for a fluorescence signal emitted by PCR reaction products to be detected, a background signal that is a fluorescence signal of a reaction sample itself and a fluorescence signal of the measurement system itself rather than a fluorescence signal caused by amplification of a target analyte accounts for most of the fluorescence signal in the baseline region.

Various methods have been developed to determine whether amplification of a target analyte is present from a real-time PCR data set.

For example, there is a method using a threshold value. The method of using a threshold value predetermines a defined signal threshold value for all reactions to be analyzed and determines whether a signal value of a data set reaches or exceeds the signal threshold value.

As another method, U.S. Patent No. 8,560,247 by Jeffrey Lerner discloses a method of obtaining a function that is fit to data and determining whether a jump error is present in a data set according to whether the slope of the obtained function exceeds a maximum amplification slope bound.

As another method, U.S. Patent Publication No. 2009-0119020 by Ronald T. Kurnik et al. discloses a method of determining whether data on a growth curve such as a PCR curve indicates valid or significant growth. The method fits data and a quadratic function to obtain a fitted quadratic function, obtains a statistically significant value (e.g., R-square value) of the quadratic function, and then determines whether the statistically significant value exceeds a certain predetermined threshold value (e.g., 0.90, 0.99).

### DETAILED DESCRIPTION OF INVENTION

### TECHNICAL PROBLEMS

An object of the present disclosure is to provide a technology for more accurately and efficiently determining whether a sample contains the above-described target nucleic acid, that is, whether it is positive or negative.

However, the object of the present disclosure is not limited to the mentioned above, and other objects that are not mentioned can be clearly understood by those skilled in the art from the description below.

### TECHNICAL SOLUTION

A positive/negative determining apparatus according to an embodiment includes: an input unit that receives signal values from a signal-generating reaction of a plurality of cycles targeting a target analyte, wherein the signal-generating reaction generates a signal dependent on a presence of the target analyte in a sample; and a positive/negative determining unit pre-trained to estimate presence/absence of the target analyte in the sample from the signal values in the plurality of cycles and a sequence of the plurality of cycles.

Further, the target analyte may include a target nucleic acid molecule.

Further, the signal dependent on the presence of the target analyte may include fluorescence signal.

Further, the signal-generating reaction may include nucleic acid amplification reaction.

Further, the positive/negative determining unit may obtain the signal value from the input unit sequentially according to the sequence of the cycles

Further, the positive/negative determining unit may estimate the presence/absence of the target analyte in the sample after obtaining all of the signal values of the plurality of cycles from the input unit.

Further, in a training process of the positive/negative determining unit, training data including training input data and training correct answer data may be used, the training input data may include signal values for a plurality of training cycles, which are previously obtained by the signal generation reaction for generating a signal dependent on the presence of the target analyte, and information about a sequence of the training cycles, and the training correct answer data may include information on the presence/ absence of the target analytes.

Further, in the training process of the positive/negative determining unit, the signal values for the plurality of training cycles among the training input data may be sequentially input to the positive/negative determining unit according to the sequence of the cycles.

Further, the training process of the positive/negative determining unit may include: comparing a result estimated in response to the training input data input to the positive/negative determining unit and the training correct answer data; and training the positive/negative determining unit to reduce a difference derived through the comparison.

Further, in the process of training the positive/negative determining unit to reduce the difference, a backpropagation method may be used

Further, when the positive/negative determining unit includes a BERT (Bidirectional Encoder Representations from Transformers) model, the positive/negative determining unit may include: a body part that is trained based at least in part on the training input data; and a fine-tuning part that is combined with the trained body part, and is trained to infer whether the training input data is positive or negative when the training input data is input to the trained body part.

Further, the body part is trained according to a semi-supervised learning method.

Further, the semi-supervised learning may include: masking a part of the training input data and then matching the masked part; and matching an order or a precedence relation of signal values having an order included in the training input data.

Further, the fine-tuning part may be trained according to a supervised-learning method.

Further, the positive/negative determining unit may include: an encoder part that generates a context vector from the signal values in the plurality of cycles and the sequence of each cycle; and a binary classification part that estimates the presence/absence of the target analyte in the sample from the context vector.

Further, in a training process when the positive/negative determining unit includes a recurrent neural network model or a long short-term memory model, the positive/negative determining unit may be trained such that a weight for a signal value of any one cycle among the plurality of cycles and a weight for a hidden state value calculated for a cycle that temporally precedes the one cycle are updated so that a difference between a result output from an output terminal of the positive/negative determining unit in response to training input data input to an input terminal of the positive/negative determining unit and training correct answer data is reduced.

Further, an input/output structure of the positive/negative determining unit when the positive/negative determining unit may include a recurrent neural network model or a long short-term memory model is a many-to-one structure that outputs one data when receiving multiple data.

Further, the positive/negative determining unit may be provided in the positive/negative determining apparatus for each predefined factor that affects a signal generation of the signal-generating reaction.

Further, the predefined factor may include reaction conditions in a nucleic acid amplification reaction.

Further, the reaction conditions may be a reaction medium used for the nucleic acid amplification reaction.

Further, the reaction medium may be one or more substances selected from a group consisting of pH-related substances, ionic strength-related substances, enzymes and enzyme stabilization-related substances

Further, the pH-related substances may include buffer, the ionic strength-related substances may include ionic substances, and the enzyme stabilization-related substances may include sugar.

Further, the apparatus may further include a pre-processor that performs a predetermined pre-processing operation on the signal value in each of the plurality of cycles, wherein the positive/negative determining unit estimates the presence/absence of the target analyte in the sample from the signal value on which the pre-processing operation is performed by the pre-processor.

Further, the pre-processing operation may include a correction operation for an abnormal signal among the signal values in the plurality of cycles, a smoothing operation for the signal values in the plurality of cycles, or a baseline subtraction operation for subtracting a baseline signal value regressed from the signal values in the plurality of cycles from the signal values in the plurality of cycles.

A positive/negative determining method executed by a positive/negative determining apparatus according to an embodiment, including: receiving signal values from a signal-generating reaction of a plurality of cycles targeting a target analyte, wherein the signal-generating reaction generates a signal dependent on a presence of the target analyte in a sample; and estimating presence/absence of the target analyte in the sample from the signal values in the plurality of cycles and a sequence of the plurality of cycles by a pre-trained model.

A computer-readable storage medium storing a computer program according to an embodiment, wherein the computer program includes instructions for causing a processor to perform a positive/negative determining method targeting a target analyte executed by a positive/negative determining apparatus, and wherein the method includes: receiving signal values from a signal-generating reaction of a plurality of cycles targeting a target analyte, wherein the signal-generating reaction generates a signal dependent on a presence of the target analyte in a sample; and estimating presence/absence of the target analyte in the sample from the signal values in the a plurality of cycles and a sequence of the a plurality of cycles by a pre-trained model.

### EFFECT OF INVENTION

According to one embodiment, whether a sample is positive or negative for a target analyte can be determined by a model trained using a deep learning method. Accordingly, the rapidity and accuracy of positive/negative determination results can be improved.

### BRIEF DESCRIPTION OF THE DRAWING

FIG. 1 is a block diagram of a positive/negative determination system including a positive/negative determining apparatus, a preparation apparatus, and a detection apparatus according to an embodiment.
FIG. 2 is a conceptual diagram of the positive/negative determining apparatus according to an embodiment.
FIG. 3 is a block diagram showing a configuration of the positive/negative determining apparatus according to an embodiment.
FIG. 4 is a conceptual diagram showing the concept of a recurrent neural network model.
FIG. 5 is a conceptual diagram showing a case in which a positive/negative determining apparatus according to an embodiment is implemented with the recurrent neural network.
FIG. 6 is a hardware block diagram of the positive/negative determining apparatus according to an embodiment.
FIG. 7 is a flowchart of a positive/negative determining method according to an embodiment.
FIGS. 8A to 8F are graphs showing signal values at each temperature in a plurality of cycles of a sample when at least one target analyte is contained in the sample according to an embodiment.

### BEST MODE FOR CARRYING OUT THE INVENTION

The advantages and features of the present disclosure and methods for achieving the same will become clear by referring to embodiments described in detail below along with the accompanying drawings. However, the present disclosure is not limited to the embodiments disclosed below and may be implemented in various different forms. The present embodiments are merely provided to ensure that the present disclosure is complete and to fully inform those skilled in the art in the technical field to which the present invention pertains of the scope of the present disclosure, and the present disclosure is only defined by the scope of the claims.

In describing embodiments of the present disclosure, if it is determined that a detailed description of a known function or configuration may unnecessarily obscure the gist of the present disclosure, the detailed description will be omitted. The terms described below are defined in consideration of functions in the embodiments of the present disclosure and may vary depending on the intention or custom of the user or operator. Therefore, the definition should be made based on the content throughout the specification.

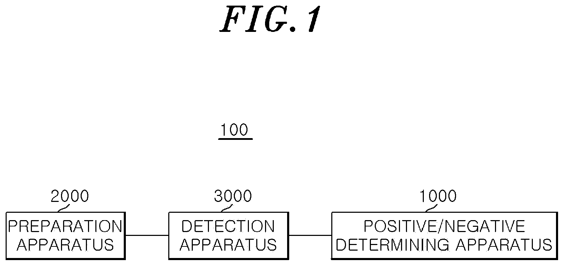

Prior to describing FIG. 1, the terms used herein will be described.

The term "target analyte" includes a variety of substances (e.g., biological and non-biological substances), which may refer to the same entity as the term "target analytical substance."

Such a target analyte specifically includes a biological substance, more specifically, at least one of nucleic acid molecules (e.g., DNA and RNA), proteins, peptides, carbohydrates, lipids, amino acids, biological compounds, hormones, antibodies, antigens, metabolites, and cells.

The term "sample" includes biological samples (e.g., cells, tissues, and body fluids) and non-biological samples (e.g., food, water, and soil). Among these, the biological samples include, for example, at least one of viruses, bacteria, tissues, cells, blood (including whole blood, plasma and serum), lymph, bone marrow fluid, saliva, sputum, swab, aspiration, milk, urine, stool, eye fluid, semen, brain extract, spinal fluid, joint fluid, thymic fluid, bronchial lavage fluid, ascites, and amniotic fluid. These samples may or may not contain the target analytes described above.

Meanwhile, when the above-mentioned target analyte is a nucleic acid molecule or contains a nucleic acid molecule, a nucleic acid extraction process known in the art may be performed on the sample estimated to contain the target analyte (Reference: Sambrook, J. et al., Molecular Cloning. A Laboratory Manual, 3rd ed. Cold Spring Harbor Press (2001)). The nucleic acid extraction process may vary depending on the type of sample. In addition, if the extracted nucleic acid is RNA, it can be additionally subjected to a reverse transcription process to synthesize cDNA (Reference: Sambrook, J. et al., Molecular Cloning. A Laboratory Manual, 3rd ed. Cold Spring Harbor Press (2001)).

The term "data set" refers to data obtained from a signal-generating reaction for the target analyte using a signal generation means (the signal generation means will be described below).

In this case, the term "signal-generating reaction" refers to a reaction that generates a signal depending on the properties of the target analyte, such as activity, amount, or presence (or absence) of the target analyte in the sample, specifically the presence (or absence). Such a signal-generating reaction includes biological reactions and chemical reactions. Among these, biological reactions include genetic analysis processes such as PCR, real-time PCR, and microarray analysis, immunological analysis processes, and bacterial growth analysis. In addition, chemical reactions include a process of analyzing creation, change, or destruction of a chemical substance. According to one implementation example, the signal-generating reaction may be a genetic analysis process, or may be a nucleic acid amplification reaction, an enzyme reaction, or microbial growth.

Meanwhile, the signal-generating reaction described above is accompanied by signal change. Therefore, the progress of this signal-generating reaction can be evaluated by measuring a change in a signal.

Here, the term "signal" refers to a measurable output. In addition, the measured magnitude or change of the signal serves as an indicator qualitatively or quantitatively indicating properties of a target analyte, specifically, the presence or absence of the target analyte in a sample.

Here, examples of indicators include fluorescence intensity, luminescence intensity, chemiluminescence intensity, bioluminescence intensity, phosphorescence intensity, charge transfer, voltage, current, power, energy, temperature, viscosity, light scatter, radioactivity intensity, reflectivity, light transmittance, and absorbance, but are not limited thereto.

The term "signal generation means" previously described refers to a means for providing a signal indicating properties, specifically, the presence or absence, of a target analyte to be analyzed.

The term "signal generation means" may be represented as "signal generating composition."

Such a signal generation means includes a label itself or an oligonucleotide to which the label is linked.

Among these, labels include fluorescent labels, luminescent labels, chemiluminescent labels, electrochemical labels, and metal labels. The label may be used as a label itself, such as an intercalating dye. Alternatively, the label may be used in the form of a single label or an interactive dual label including a donor molecule and an acceptor molecule, bound to one or more oligonucleotides.

The signal generation means may additionally include an enzyme having nucleic acid cleavage activity to generate a signal (for example, an enzyme having 5' nucleic acid cleavage activity or an enzyme having 3' nucleic acid cleavage activity).

Meanwhile, various methods for generating a signal indicating the presence of a target analyte, particularly a target nucleic acid molecule, using the signal generation means are known. Representative examples may include: TaqManTM probe method (U.S. Patent No. 5,210,015); molecular beacon method (Tyagi, Nature Biotechnology, v.14 MARCH 1996); Scorpion method (Whitcombe et al., Nature Biotechnology 17:804-807 (1999)); Sunrise (or Amplifluor) method (Nazarenko et al., Nucleic Acids Research, 25(12):2516-2521 (1997), and US Patent No. 6,117,635); Lux method (U.S. Patent No. 7,537,886); CPT (Duck P, et al. Biotechniques, 9:142-148 (1990)); LNA method (U.S. Patent No. 6,977,295); Plexor method (Sherrill CB, et al., Journal of the American Chemical Society, 126:4550-4556 (2004)); Hybeacons (D.J. French, et al., Molecular and Cellular Probes 13:363-374 (2001) and U.S. Patent No. 7,348,141); dual-labeled self-quenched probe (U.S. Patent No. 5,876,930); hybridization probe (Bernard PS, et al., Clin Chem 2000, 46, 147-148); PTOCE (PTO cleavage and extension) method (WO 2012/096523); PCE-SH (PTO Cleavage and Extension-Dependent Signaling Oligonucleotide Hybridization) method (WO2013/115442); PCE-NH (PTO Cleavage and Extension-Dependent Non-Hybridization) method (PCT/KR2013/012312); and CER method (WO 2011/037306).

Meanwhile, the aforementioned term "signal-generating reaction" may include a signal amplification reaction. At this time, the term "amplification reaction" refers to a reaction that increases or decreases a signal generated by the signal generation means. Specifically, the amplification reaction refers to a signal increase (or amplification) reaction generated by the signal generation means depending on the presence of a target analyte.

In this amplification reaction, amplification of a target analyte (e.g., nucleic acid molecule) may or may not be accompanied. More specifically, the amplification reaction may refer to a signal amplification reaction accompanied by amplification of a target analyte.

Meanwhile, a data set obtained through the amplification reaction may include an amplification cycle.

Here, the term "cycle" refers to a unit of change in certain conditions in a plurality of measurements involving changes in the conditions. Changes in the aforementioned certain conditions mean, for example, increase or decrease in temperature, reaction time, the number of reactions, concentration, pH, the number of copies of a measurement target (e.g., nucleic acid), and the like. Accordingly, a cycle can be a time or process cycle, a unit operation cycle, and a reproductive cycle.

More specifically, the term "cycle" refers to one unit of repetition when a certain process of reaction is repeated or a reaction is repeated at a certain time interval.

For example, in the case of a nucleic acid amplification reaction, one cycle means a reaction including denaturation of a nucleic acid, annealing of a primer, and extension of the primer. In this case, a change in certain conditions is an increase in the number of repetitions of the reaction, and the repetition unit of the reaction including the series of steps is set as one cycle.

Meanwhile, an amplification reaction for amplifying a signal indicating the presence of a target analytical substance (target analyte) may be performed in a manner in which the signal is also amplified while the target analyte is amplified (e.g., real-time PCR method). Alternatively, according to one implementation example, the amplification reaction may be performed in a manner in which the target analyte is not amplified and only the signal indicating the presence of the target analyte is amplified (e.g., CPT method (Duck P, et al., Biotechniques, 9:142-148 (1990)), Invader assay (U.S. Patent Nos. 6,358,691 and 6,194,149)).

Meanwhile, the above-described target analytical substance or target analyte, particularly a target nucleic acid molecule, can be amplified by various methods: polymerase chain reaction (PCR); ligase chain reaction (LCR) (U.S. Patent Nos. 4,683,195 and 4,683,202; PCR Protocols: A Guide to Methods and Applications (Innis et al., eds, 1990)); strand displacement amplification (SDA) (Walker, et al. Nucleic Acids Res. 20(7):1691-6 (1992); Walker PCR Methods Appl 3(1): 1-6 (1993)); transcription-mediated amplification (Phyffer, et al., J. Clin. Microbiol. 34:834-841 (1996); Vuorinen, et al., J. Clin. Microbiol. 33: 1856-1859 (1995)); nucleic acid sequence-based amplification (NASBA) (Compton, Nature 350(6313):91-2 (1991)); rolling circle amplification (RCA) (Lisby, Mol. Biotechnol. 12(1):75-99 (1999); Hatchet et al., Genet Anal. 15(2):35-40 (1999)); and Q-Beta Replicase (Lizardi et al., BiolTechnology 6:1197 (1988)).

Meanwhile, the amplification reaction amplifies a signal while amplifying a target analyte (specifically, a target nucleic acid molecule). For example, the amplification reaction is carried out according to PCR, specifically real-time PCR, or isothermal amplification reaction (e.g., LAMP or RPA).

Meanwhile, a data set obtained by a signal-generating reaction includes a plurality of data points including cycles of the signal-generating reaction and signal values at the cycles.

Here, the term "signal value" refers to a value obtained by quantifying the level (e.g., signal intensity) of a signal actually measured in a cycle of the signal-generating reaction, especially the amplification reaction, according to a certain scale or a modified value thereof. The modified value may include a mathematically processed signal value of the actually measured signal value. Examples of a mathematically processed signal value of an actually measured signal value (i.e., a signal value of a raw data set) may include logarithms or derivatives.

The term "data point" refers to a coordinate value including a cycle and a signal value. Additionally, the term "data" refers to all information constituting a data set. For example, a cycle and a signal value of an amplification reaction may correspond to data.

Data points obtained by a signal-generating reaction, especially an amplification reaction, can be represented as coordinate values that can be expressed in a two-dimensional orthogonal coordinate system. In the coordinate values, the X-axis represents the number of cycles, and the Y-axis represents signal values measured or processed in the cycles.

The term "data set" refers to a set of data points. For example, the data set may be a set of data points obtained directly through an amplification reaction performed in the presence of a signal generation means, or may be a modified data set obtained by modifying such a data set. The data set may be some or all of a plurality of data points obtained by an amplification reaction or modified data points thereof.

According to one implementation example, the data set for the target analyte includes 200 or less, 150 or less, 100 or less, 80 or less, 60 or less, 50 or less, 45 or less, 40 or less, and 30 or less data points.

According to one implementation example, a data set for a target analyte includes two or more, 5 or more, 10 or more, 15 or more, and 20 or more data points.

Meanwhile, a data set may be a data set obtained by processing a plurality of data sets. When analysis of a plurality of target analytes is performed in one reaction vessel, a data set for the plurality of target analytes may be obtained through processing of data sets obtained from reactions performed in the single reaction vessel in some cases. For example, a data set for a plurality of target analytes analyzed in one reaction vessel can be obtained by processing a plurality of data sets obtained from signals measured at different temperatures.

The above-described data set may be plotted, thereby obtaining an amplification curve.

With reference to the drawings below, various implementation examples of the present disclosure will be described.

FIG. 1 is a block diagram of a positive/negative determination system 100 including a positive/negative determining apparatus 1000, a preparation apparatus 2000, and a detection apparatus 3000 according to an embodiment.

Referring to FIG. 1, the positive/negative determination system 100 includes the positive/negative determining apparatus 1000, the preparation apparatus 2000, and the detection apparatus 3000. Additionally, these components may be connected to each other by wired or wireless communication.

However, the block diagram shown in FIG. 1 is merely illustrative, and the spirit of the present disclosure is not limited to that shown in FIG. 1. For example, the positive/negative determination system 100 may additionally include components that are not shown in FIG. 1 or may not include at least one of the components shown in FIG. 1.

The positive/negative determination system 100 may represent only the positive/negative determining apparatus.

Additionally, the components of the positive/negative determination system 100 may be connected in a different manner from that shown in FIG. 1. Hereinafter, each configuration will be described in detail.

First, the preparation apparatus 2000 is implemented to perform a preparation operation on a sample (or an analysis sample).

Here, the above-described preparation operation performed by the preparation apparatus 2000 includes a nucleic acid extraction operation and an operation of preparation of reaction mixture for nucleic acid amplification. However, depending on an embodiment, the preparation apparatus 2000 may not perform the nucleic acid extraction operation. In this case, the nucleic acid extraction operation may be performed by another component that is not shown in FIG. 1.

The detection apparatus 3000 is implemented to perform a nucleic acid amplification reaction and a nucleic acid detection operation on a sample. Depending on an embodiment, the detection apparatus 3000 may be implemented to perform a nucleic acid detection operation without performing a nucleic acid amplification reaction. However, the following description will be made on the assumption that the detection apparatus 3000 is implemented to also perform a nucleic acid amplification reaction.

The detection apparatus 3000 may include an optical module and a thermal module.

The optical module includes a light source module and a detection module. The light source module supplies appropriate optical stimulation to a sample, and the detection module detects an optical signal generated from the sample in response. The optical signal may be luminescence, phosphorescence, chemiluminescence, fluorescence, polarized fluorescence, or other colored signals. The optical signal may be an optical signal generated in response to optical stimulation applied to the sample. The light source module includes a light source configured to emit light to a sample and a filter that filters light emitted from the light source. The light source may be a light source that emits light having the same wavelength characteristics. For example, if light sources emit light in the same wavelength range, it means that the amounts of light emitted for respective wavelength ranges are identical. "Identical" means not only being completely identical but also being substantially identical. Substantially identical means that, for example, when light generated from two light sources is radiated to the same optical label through the same filter, the same type of emitted light is generated at the same level and in the same amount of light from the optical label. Specifically, the fact that a plurality of light sources has substantially the same wavelength characteristics means that the deviation of the amounts of light or wavelength ranges of the plurality of light sources is within 20%, 15%, or 10%.

The detection module detects a signal. The detection module detects fluorescence, which is an optical signal generated from samples.

The detection module detects an optical signal by generating an electrical signal according to the intensity of the optical signal.

The detection module includes a detector configured to detect light emitted from a sample and a filter that filters the light emitted from the sample.

The detector is configured to detect light emitted from an optical label included in a sample. The detector may detect the amount of light for each wavelength by distinguishing wavelengths of light, or may detect the total amount of light regardless of wavelengths. Specifically, the detector may use, for example, a photodiode, a photo diode array, a photo multiplier tube (PMT), a CCD image sensor, a CMOS image sensor, an avalanche photodiode (APD), or the like.

The detector is configured to detect light emitted from the optical label included in the sample.

Specifically, the detector may be formed toward the sample such that emitted light generated from the sample can directly reach the detector or toward a reflector or an optical fiber such that the emitted light can reach the detector through the reflector or the optical fiber.

The filter of the detection module is a filter that selectively passes the light emitted from the optical label included in the sample. If light in a wavelength range other than the light emitted from the optical label included in the sample is detected by the detector, an optical signal cannot be accurately detected. The detection filter of the present disclosure allows a target to be accurately detected by selectively passing the light emitted from the optical label.

The thermal module is implemented to perform a nucleic acid amplification reaction. For this purpose, the thermal module includes a sample holder in which a sample is accommodated. The sample holder includes a plurality of holes, and reaction vessels may be located in the holes. Reaction vessels can contain samples, and fluorescence is emitted from the samples. The sample holder may be a conductive material. When the sample holder contacts the reaction vessels, heat may be transferred from the sample holder to the reaction vessels. For example, the sample holder can be made of metal such as aluminum, gold, silver, nickel, or copper. Alternatively, a separate component other than the sample holder may directly supply energy to the reaction vessels to control the temperature of the samples in the reaction vessels. In this case, the sample holder may be configured such that it accommodates the reaction vessels but does not transfer heat to the reaction vessels.

One example of the sample holder is a thermal block. The thermal block includes a plurality of holes, and reaction vessels may be located in the holes.

Another example of the sample holder is a heating plate. The heating plate is made by contacting a thin metal plate with a sample. The heating plate can operate in such a manner that it is heated by passing an electric current through the thin metal.

Another example of the sample holder is a receiving part that can accommodate one or more chips or cartridges. An example of a cartridge is a fluid cartridge including a flow channel.

The thermal module may additionally include a heat generating element, a heat sink, and a heat sink fan for circulating samples in the sample holder through a series of temperatures.

Specifically, the thermal module is used for PCR-based nucleic acid amplification reactions. More specifically, the thermal module can perform a denaturing step, an annealing step, and an extension (or amplification) step to amplify deoxyribonucleic acid (DNA) having a specific nucleotide sequence.

Among the steps, the denaturation step is a step in which double-stranded DNA is separated into single-stranded DNA by heating a solution containing a sample containing the double-stranded DNA, which is a template nucleic acid, and a reagent to a specific temperature, for example, about 95°C. The annealing step is a step in which an oligonucleotide primer having a nucleotide sequence complementary to the nucleotide sequence of the nucleic acid to be amplified is provided and cooled together with the separated single-stranded DNA to a specific melting temperature Tm, for example, 60°C, to bind the primer to a specific nucleotide sequence of the single-stranded DNA, thereby forming a partial DNA-primer complex. The extension step is a step in which double-stranded DNA is formed based on the primer of the partial DNA-primer complex by DNA polymerase by maintaining the solution at a specific temperature, for example, 72°C, after the annealing step.

In one embodiment, the thermal module can exponentially amplify DNA having the specific nucleotide sequence by repeating the three steps described above, for example, 10 to 50 times.

In another embodiment, the thermal module may perform the annealing step and the extension step simultaneously. In this case, the thermal module may complete one cycle (circulation) by performing two steps consisting of the denaturation step and the annealing/extension step.

The detection apparatus includes various devices known in the art as long as they can control temperature for the amplification reaction. Examples thereof include CFX (Bio-Rad), iCycler (Bio-Rad), LightCycler (Roche), StepOne (ABI), 7500 (ABI), ViiA7 (ABI), QuantStudio (ABI), AriaMx (Agilent), Eco (Illumina), etc., but are not limited thereto. Meanwhile, if a target analyte is contained in a reaction vessel, the amount is amplified as the above-described nucleic acid amplification reaction is performed, and the magnitude of a signal generated by the aforementioned signal generation means may also be amplified. Accordingly, the detection apparatus 3000 is implemented to detect the magnitude of such a signal. Specifically, the detection apparatus 3000 can monitor the magnitude of the above-described signal that changes with the progress of the nucleic acid amplification reaction in real time. The monitored results may be output from the detection apparatus 3000 in the form of a data set as mentioned in the definition of terms.

Meanwhile, since the magnitude of the signal generated by the signal generation means can also be amplified when the nucleic acid amplification reaction is performed, the nucleic acid amplification reaction will be regarded hereinafter as causing the signal-generating reaction described above.

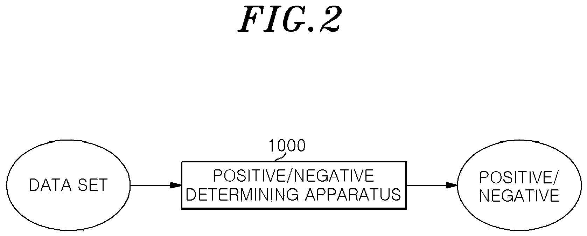

The positive/negative determining apparatus 1000 may receive a detection result with respect to a target analyte of a sample from the detection apparatus 3000. For example, the positive/negative determining apparatus 1000 may receive the previously described data set from the detection apparatus 3000. Then, the positive/negative determining apparatus 1000 can determine and output whether the sample is positive or negative for the target analyte. This is conceptually shown in FIG. 2. Referring to FIG. 2, when the positive/negative determining apparatus 1000 receives a data set as a result of detection of the target analyte of the sample, the positive/negative determining apparatus 1000 determines whether the sample is positive or negative for target analyte and outputs the determination result in response.

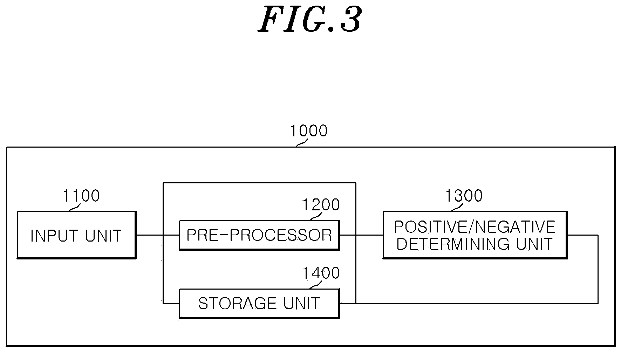

Hereinafter, each functional component of the positive/negative determining apparatus 1000 will be described in more detail with reference to FIG. 3.

FIG. 3 is a block diagram of the positive/negative determining apparatus 1000 according to an embodiment.

Referring to FIG. 3, the positive/negative determining apparatus 1000 includes an input unit 1100, a pre-processor 1200, and a positive/negative determining unit 1300, but is not limited thereto. For example, depending on an embodiment, the positive/negative determining apparatus 1000 may be implemented not to include at least one component (e.g., the pre-processor 1200) shown in FIG. 3, or may be implemented to additionally include a component that is not shown in FIG. 3.

First, the input unit 1100 is a component that receives data. To this end, the input unit 1100 may be implemented as an input/output port or terminal.

Through this input unit 1100, the positive/negative determining apparatus 1000 can receive the above-described data set from the detection apparatus 3000.

Here, as described in the definition of terms, the data set may include not only a signal value at each cycle detected in the amplification process, but also the order of the signal value for cycles, but is limited thereto.

For example, a data set may include data points paired with numbers of responses and signal values, and the data points can be arranged in ascending order of numbers of responses or descending order of numbers of responses.

For example, a data set includes data points paired with response times and signal values, and the data points can be arranged ascending order of response times or descending order of response times.

Here, the unit of a signal value may be, for example, relative fluorescence units (RFU), which represents the intensity of a fluorescence signal. In addition, the range of the cycle order may be 1 to 45, but is not limited thereto.

The pre-processor 1200 may perform a predetermined pre-processing operation on the data set received by the positive/negative determining apparatus 1000 from the detection apparatus 1300 through the input unit 1100. In this pre-processing operation, unwanted non-analyte related signals that may be generated regardless of the presence or absence of a target analyte in a sample can be removed or corrected. For example, through the aforementioned pre-processing operation, electrical noise that may be generated in the detection apparatus 3000 regardless of whether the aforementioned signal-generating reaction is executed, noise in the baseline region of the nucleic acid amplification curve, or a sudden increase in a fluorescence signal (i.e., jump, spike, or step signal) or a sudden decrease (i.e., dip signal) can be removed or corrected.

Specific methods of such a pre-processing operation include negative control subtraction, baseline subtraction, and the like.

Among these methods, the negative control subtraction will be described first. A negative control group is an experimental group in which one or more essential components for the reaction to proceed in reactants for the signal-generating reaction are missing. For example, in a nucleic acid amplification reaction, a negative control group is an experimental group without a target analyte, an experimental group without primers, an experimental group without polymerase, or an experimental group with only a buffer solution. Specifically, a negative control group used in one embodiment is an experimental group containing components for nucleic acid amplification excluding a target analyte from nucleic acid amplification reactants.

Negative control subtraction can be performed by subtracting a signal value of a negative control group from a signal value of a data set for a target analyte. Specifically, the signal value of the data set for the target analyte and the signal value of the negative control group are obtained in the same reaction and measurement environment, for example, in the same reaction run in the same amplification and analysis apparatus.

Next, baseline subtraction can be performed by various methods known in the art (e.g., U.S. Patent No. 8,560,247).

In addition to the above-described negative control subtraction or baseline subtraction, there are various types of pre-processing operations, and in one embodiment, these various types of pre-processing operations may be performed in the pre-processor 1200. For example, as a pre-processing operation, the method disclosed in U.S. Patent No. 8,560,247 or U.S. Application Publication No. 2015/0186598 may be applied.

In addition, if a signal detected from one analytical sample vessel (reaction tube) is likely to include signals for detecting a plurality of target analytes using a single type of label, the pre-processing operation may include an operation for separating or distinguishing signals with respect to the plurality of target analytes from the signal, which will be described later.

On the other hand, the aforementioned pre-processing operation may include an operation for allowing learning results with respect to the positive/negative determining unit 1300 to reach a desired level. Specifically, the positive/negative determining unit 1300 is a neural network, and thus the quality of learning data used for learning may affect the performance of the positive/negative determining unit 1300. Accordingly, a pre-processing process such as standardization or normalization targeting learning data, a pre-processing process such as detecting outliers and correcting or removing them, a pre-processing process for clustering learning data having the same or similar properties and then performing learning by taking this into account, or a pre-processing process for unifying the units of learning data may be performed as the aforementioned pre-processing operation. However, since such pre-processing processes are already known, detailed description thereof will be omitted.

The positive/negative determining unit 1300 is implemented to determine the presence or absence of a target analyte in a sample. To this end, the positive/negative determining unit 1300 may receive the above-described data set from the input unit 1100 or the pre-processor 1200.

The positive/negative determining unit 1300 can be implemented in various manners.

For example, the positive/negative determining unit 1300 can be implemented using a neural network. In this case, information input to the positive/negative determining unit 1300, that is, the data set, is input to an input terminal of the neural network, and information output from the positive/negative determining unit 1300 is a result of inference from the neural network. Among the information, the information output from the positive/negative determining unit 1300 includes an inference result regarding the presence or absence of the target analyte in the sample. If the data output in this way is "positive", it means that the sample contains the target analyte. On the other hand, if the output data is "negative", it means that the sample does not contain the target analyte.

Here, the term "neural network" may be used interchangeably with a classifier, a computational model, a neural network model, or a network function. In addition, the aforementioned terms, including the neural network, may be included within the category of machine learning models.

The neural network will be described in more detail. A neural network can be composed of a set of interconnected computational units, which can be referred to as nodes, in general. These nodes may also be referred to as neurons. The neural network includes one or more of these nodes. In addition, such nodes (or neurons) constituting neural networks may be interconnected by one or more links.

Within a neural network, one or more nodes connected through a link may form a relative relationship between an input node and an output node. The concepts of an input node and an output node are relative, and thus any node in an output node relationship with one node may be in an input node relationship with another node, and vice versa. As described above, a relationship between an input node and an output node can be created around a link. One or more output nodes can be connected to one input node through a link, and vice versa.

In a relationship between an input node and an output node connected through one link, the value of data of the output node may be determined based on data input to the input node. Here, the link connecting the input node and the output node may have a weight. Weights may be variable and may be varied by a user or an algorithm in order for the neural network to perform a desired function. For example, when one or more input nodes are connected to one output node by respective links, the output node can determine an output node value on the basis of values input to the input nodes connected to the output node and weights set for the links corresponding to the input nodes.

As described above, in a neural network, one or more nodes are interconnected through one or more links to form an input node and output node relationship within the neural network. The properties of the neural network can be determined according to the number of nodes and links within the neural network, the correlation between the nodes and links, and the value of a weight assigned to each link. For example, if the same number of nodes and links are present and two neural networks with different weight values of links are present, the two neural networks may be recognized as different from each other.

A neural network may be composed of a set of one or more nodes. A subset of nodes constituting the neural network can constitute a layer. Some of the nodes constituting the neural network may form one layer based on distances from the first input node. For example, a set of nodes with a distance n from the first input node may constitute n layers. The distance from the first input node can be defined by the minimum number of links that must be passed to reach the corresponding node from the first input node. However, this definition of a layer is arbitrary for explanation purposes, and the order of a layer within a neural network may be defined in a different way than described above. For example, a layer of nodes may be defined by a distance from the final output node.

The first input node may refer to one or more nodes through which data is directly input without going through links in relationships with other nodes among nodes within a neural network. Alternatively, the first input node may refer to nodes that do not have other input nodes connected by links in a relationship between nodes based on links in a neural network. Similarly, the final output node may refer to one or more nodes that do not have an output node in relationships with other nodes among nodes within a neural network. Additionally, hidden nodes may refer to nodes constituting a neural network other than the first input node and the last output node.

Meanwhile, in the case of a neural network according to one embodiment, the number of nodes of an input layer may be greater than the number of nodes of an output layer. For example, the number of nodes of the input layer may be 45, whereas the number of nodes of the output layer may be 2. In this case, the input layer nodes may be data points included in the above-mentioned data set, and one of two nodes of the output layer in this case may be with respect to "positive" and the other may be with respect to "negative". When the number of nodes of the output layer is two, as described above, one may correspond to "positive" indicating inclusion of a target analyte in a sample, and the other may correspond to "negative".

In the case of a neural network according to another embodiment, the number of nodes of the input layer may be less than or equal to the number of nodes of the output layer.

Meanwhile, among the aforementioned neural networks, a "deep neural network" (DNN) may refer to a neural network that includes a plurality of hidden layers in addition to the input layer and the output layer. Deep neural networks can be used to ascertain latent structures of data. That is, it is possible to ascertain latent structures of a photo, text, video, speech, and music (e.g., what object is in the photo, what the content and emotion of the text are, what the content and emotion of the speech are, etc.). Deep neural networks may include a convolutional neural network (CNN), a recurrent neural network (RNN), a Long Short-Term Memory, an auto encoder, a generative adversarial network (GAN), a restricted Boltzmann machine (RBM), a deep belief network (DBN), a Q network, a U network, a Siamese network, a generative adversarial network (GAN), bidirectional encoder representations from transformers (BERT) model, and the like. However, the description of the deep neural networks described above is merely an example and the present disclosure is not limited thereto.

Meanwhile, the neural network according to one embodiment may be trained using at least one of supervised learning, unsupervised learning, semi-supervised learning, and reinforcement learning.

Training of such a neural network may be a process of applying knowledge for the neural network to perform a specific operation to the neural network. Details of training and learning data of the neural network which will be described later may be about training or learning data of the positive/negative determining unit 1300 of the present disclosure.

Neural networks can be trained to minimize output errors. In training a neural network, learning data is repeatedly input to the neural network, the output of the neural network and the error of a target with respect to the learning data are calculated, and the error of the neural network is backpropagated from the output layer of the neural network to the input layer in a direction of reducing the error to update the weight of each node in the neural network. In the case of supervised learning, learning data in which a correct answer is labeled for each learning data is used (i.e., labeled learning data), and in the case of unsupervised learning, a correct answer may not be labeled for each piece of learning data. That is, for example, in the case of supervised learning on data classification, learning data may be data in which each piece of learning data is labeled with a category. Labeled learning data is input to the neural network, and an error can be calculated by comparing the output (category) of the neural network with the label of the learning data. As another example, in the case of unsupervised learning on data classification, an error can be calculated by comparing input learning data with a neural network output. The calculated error is backpropagated in the neural network in the reverse direction (i.e., from the output layer to the input layer), and a connection weight of each node in each layer of the neural network can be updated according to backpropagation. The amount of change in the connection weight of each updated node may be determined according to a learning rate. Computation of the neural network for input data and error backpropagation can constitute a learning cycle (epoch). The learning rate may be applied differently depending on the number of repetitions of the learning cycle of the neural network. For example, in the early stage of neural network training, a high learning rate can be used to ensure that the neural network rapidly achieves a certain level of performance to increase efficiency, and in the later stage of training, a low learning rate can be used to increase accuracy.

In neural network training, learning data may be a subset of actual data (i.e., data to be processed using the trained neural network) in general, and thus a learning cycle in which errors with respect to the learning data decrease but errors with respect to actual data increase may be present. Overfitting is a phenomenon in which errors in actual data increase due to excessive learning on learning data. For example, a phenomenon in which a neural network that has learned a cat by seeing a yellow cat fails to recognize that a non-yellow cat is a cat may be a type of overfitting. Overfitting can cause errors in machine learning algorithms to increase. To prevent such overfitting, various optimization methods can be used. To prevent overfitting, methods such as increasing the amount of learning data, regularization, dropout to disable some of network nodes during a learning process, and utilization of a batch normalization layer can be applied.

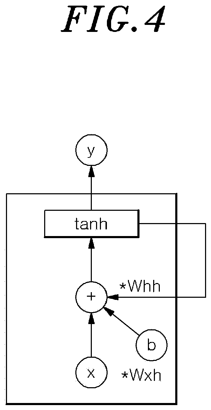

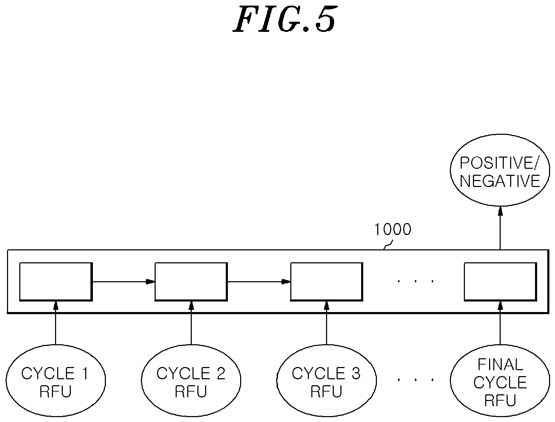

Hereinafter, a case in which the positive/negative determining unit 1300 is implemented as a neural network, especially a deep neural network, and more specifically, a recurrent neural network (RNN) will be described in more detail with reference to FIGS. 4 and 5.

A recurrent neural network is one of neural networks that can process time series data. Therefore, when the positive/negative determining unit 1300 according to an embodiment is implemented as a recurrent neural network, as a data set input to the positive/negative determining unit 1300, that is, information on signal values in a plurality of cycles and the order of the plurality of cycles originating from a signal-generating reaction of the plurality of cycles targeting a target analyte, not only the magnitudes of the signal values but also information on the order can be considered by the positive/negative determining unit 1300. For example, when there is a signal value for each of cycles 1 to 45, the positive/negative determining unit 1300 can consider a cycle in which the magnitude of the signal value increases, a cycle in which the magnitude of the signal value decreases, cycles that do not change, and cycles having a constant slope at the time of positive/negative determination.

Hereinafter, such a recurrent neural network itself will be described.

First, a method of training the recurrent neural network will be described. The recurrent neural network can be trained by a learning device (not shown in the figure) according to a supervised learning method. Here, the learning device includes a processor such as a GPU, but is not limited thereto.

Particularly in the present embodiment, the recurrent neural network outputs one piece of data when receiving a plurality of pieces of data. Among these, the input is a signal value in a plurality of cycles, and the output is whether it is positive or negative. This is shown in FIG. 5.

Next, data for training the recurrent neural network will be described. In recurrent neural network training, learning data divided into input data for learning and correct answer data for learning is used. This learning data is obtained from a signal generation response for each of a plurality of samples. Specifically, the input data for learning may include a data set obtained during a process of detecting a specific target analyte in a specific sample. In addition, the correct answer data for learning may include whether the target analyte is included in the sample, that is, whether it is positive or negative. In the current industry, numerous data sets are created and stored in the process of detecting whether various target analytes are included in each of various samples. In addition, the detection results are also generated and stored.

According to one implementation example, input data for learning used for learning in order to implement a positive determining unit used to determine whether a target analyte in a sample is positive or negative is a data set generated by a signal-generating reaction substantially the same as the signal-generating reaction used to analyze the target analyte in the sample.

Next, a learning process of the recurrent neural network will be described. As input data for learning, the above-described data set for a specific target analyte in a specific sample (e.g., a signal value in each of 45 cycles and information on the order of the cycles) is input to the recurrent neural network. As correct answer data for learning, whether the target analyte is included in the sample is input. For example, each of N pieces of learning data as shown in Table 1 below can be input into the recurrent neural network.

**[Table 1]**

| No | Input data for learning | Correct answer data for learning |
|---|---|---|
| 1 | {100, 120, 93, ......, 3000, 3100, 5000, 7000, 7500, 8232} | Negative |
| 2 | {500, 720, 1030, , 5000, 6100, 7000, 8100, 9500, 10232} | Negative |
| .. | ... | ... |
| N | {100, 123, 83, ......, 10000, 13100, 14000, 17000, 17500,18232} | Positive |

Here, in Table 1, "No" indicates a number assigned to each of the N pieces of learning data to distinguish the N pieces of learning data. However, these numbers may not be considered in recurrent neural network training described above. For example, the aforementioned N pieces of learning data may be input to the recurrent neural network in an order that is unrelated to the sizes of the numbers.

In addition, in Table 1, the input data for learning includes a plurality of signal values separated by commas "," within curly brackets " { }". In Table 1, the magnitude of these signal values may be 45, but is not limited thereto. In addition, the plurality of signal values separated by commas may be sequentially arranged from the left for the first cycle, the second cycle, .... , and the rightmost signal value may be for the last cycle (e.g., 45th cycle).

Additionally, in Table 1, the correct answer data for learning may have either "positive" or "negative" value. Here, "positive" indicates that the sample with respect to the corresponding No contains a target analyte, and "negative" indicates that the sample with respect to the corresponding No does not contain a target analyte.

Then, learning is performed in error backpropagation such that errors between the output result according to input and the aforementioned correct answer data for learning is minimized.

In such recurrent neural network training, a weight (Wₓₕ in FIG. 4) for the signal value (x in FIG. 4) of any one cycle among the plurality of cycles, and a weight (Wₕₕ in FIG. 4) for a hidden state value calculated in a cycle temporally preceding the any one cycle is learned by being updated such that the difference between the result estimated in response to the input data for learning being input to the input terminal of the positive/negative determining unit and the correct answer data for learning is reduced.

This learning process is performed until the performance of the recurrent neural network satisfies a predetermined criterion. The predetermined criterion can be determined in various ways. For example, the predetermined criterion may be determined by already known cross-validation.

Here, since the learning process of the recurrent neural network, the error backpropagation method, or the cross-validation has been previously described and are already known technology, detailed descriptions thereof will be omitted.

Meanwhile, depending on the embodiment, the positive/negative determining unit 1300 may be implemented as a Long Short-Term Memory model. The long-term and short-term memory model is known as a model that compensates for the loss of the influence of past data, which is a drawback of recurrent neural networks. Here, a learning method for the Long Short-Term Memory model and a method of use after learning is completed are the same as those of the recurrent neural network, and thus description thereof will be omitted.

Next, depending on the embodiment, the positive/negative determining unit 1300 may be implemented using the bidirectional encoder representations from transformers (BERT) model. BERT is composed of a body part that is trained using a pre-training method and a fine-tuning part that is trained using a fine-tuning method.

Here, in the body part training process, a plurality of data sets obtained when each of various samples contains various target analytes are used in semi-supervised learning known as Mask Language Model (MLM) and Next Sentence Prediction (NSP). Specifically, training according to the MLM described above can be applied to the body part through a process of masking a part of each data set and fitting the same. Additionally, each data set includes signal values in an order for each cycle, and training according to the NSP described above can be applied to the body part through a process of matching the order or sequential relationship of the signal values.

In addition, in the training process of the fine tuning part, supervised learning is performed using a feature vector derived from the body part as input and whether a sample contains a target analyte as a correct answer, and the fine tuning part is trained accordingly.

In addition, the BERT model itself is already known, except that the BERT model is used to estimate a target analyte in a sample and data used to train the BERT model is a data set and whether it is positive or negative, and thus additional description thereof will be omitted.

Meanwhile, when the above-described positive/negative determining unit 1300 is implemented using the recurrent neural network model, the Long Short-Term Memory model, or the BERT model, the positive/negative determining unit 1300 can be conceptually regarded as including an encoder part that extracts a context vector from information included in the data set, that is, the magnitudes of signal values for all cycles when the information is input, and a binary classification part that determines positive or negative based on the extracted context vector. In this regard, the positive/negative determining unit 1300 according to one embodiment may be implemented to include the encoder part and the binary classification part.

Meanwhile, the positive/negative determining unit 1300 described above may be provided in the positive/negative determining apparatus for each predefined factor that affects signal generation of the signal-generating reaction.

The factor includes reaction conditions used in nucleic acid amplification reactions.

The reaction conditions include a reaction medium used in the nucleic acid amplification reaction. The reaction medium is one or more substances selected from the group consisting of a pH-related substance, an ionic strength-related substance, an enzyme, and an enzyme stabilization-related substance.

The pH-related substance includes a buffer, the ionic strength-related substance includes an ionic material, and the enzyme stabilization-related substance includes a sugar.

For example, one positive/negative determining unit 1300 may be provided in the positive/negative determining apparatus 1000 for each target analyte such as sars-cov-2 and Zika virus. In this case, which one of a plurality of positive/negative determining units 1300 will be used may be determined by a separate algorithm or a user using the positive/negative determining apparatus 1000.

According to one implementation example, even if the target analyte is the same, the separate positive/negative determining unit may be provided depending on the signal-generating reaction used to detect the target analyte.

According to one implementation example, the positive/negative determining unit may be provided for each factor predefined as affecting signal generation of a signal-generating reaction.

According to one implementation example, the factor predefined as affecting signal generation of a signal-generating reaction includes oligonucleotides, enzymes, labels, and reaction composition component concentrations for target analyte detection.

Reaction composition components include a buffer, a salt, a metal ion, and dNTP.

According to one implementation example, the factor predefined as affecting signal generation of a signal-generating reaction includes signal-generating reaction conditions.

Oligonucleotides include primers or probes.

As described above, according to one embodiment, whether a sample is positive or negative for a target analyte can be determined by a model trained using a deep learning method. Accordingly, the rapidity and accuracy of positive/negative determination results can be improved.

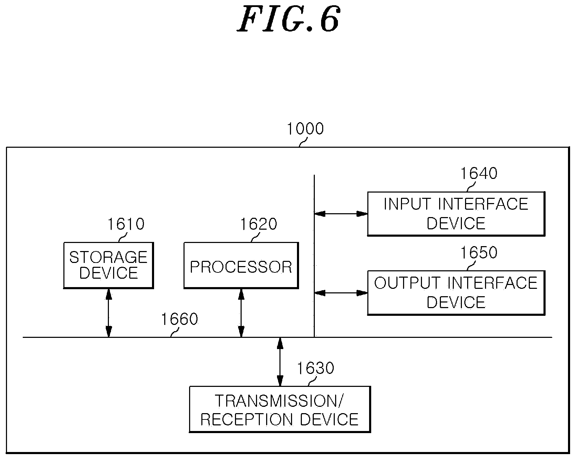

Meanwhile, the above-described positive/negative determining apparatus 1000 may be implemented according to the hardware block diagram as shown in FIG. 6. This will be described in more detail.

Referring to FIG. 6, the positive/negative determining apparatus 1000 may include a storage device 1610 that stores at least one command, a processor 1620 that executes at least one command of the storage device 1610, and a transmission/reception device 1630, an input interface device 1640, and an output interface device 1650.

The components 1610, 1620, 1630, 1640, and 1650 included in the positive/negative determining apparatus 1000 can perform communication with each other by being connected by a data bus 1660.

The storage device 1610 can perform the function of a storage unit 1400. Additionally, the storage device 1610 may include a memory or at least one of a volatile storage medium and a non-volatile storage medium. For example, the storage device 1610 may include at least one of a read only memory (ROM) and a random access memory (RAM).

The storage device 1610 may further include at least one command to be executed by the processor 1620, which will be described later, and may store signal values, learning data, and the like for each of a plurality of cycles input from the user through the input interface device 1640.

The processor 1620 may refer to a central processing unit (CPU), a graphics processing unit (GPU), a micro controller unit (MCU), or a dedicated processor through which methods according to embodiments are performed.

Referring further to FIG. 1, the processor 1620 can perform the functions of the pre-processor 1200 and the positive/negative determining unit 1300 using at least one program command stored in the storage device 1610, as described above, and each program command may be stored in a memory in the form of at least one module and executed by the processor.

The transmission/reception device 1630 can receive or transmit data from an internal device or an external device connected through communication, and can execute the function of the input unit 1100. For example, the transmission/reception device 1630 may receive data regarding samples, analysis samples, and data sets of amplified samples from the preparation apparatus 2000 or the detection apparatus 3000.

The input interface device 1640 may receive at least one control signal or setting value from the user. For example, the input interface device 1640 may receive user input, such as a pre-processing method to be performed by the pre-processor 1200 and a jumping correction criterion.

The output interface device 1650 may output and visualize at least one piece of information including whether a sample determined by the positive/negative determining unit 1300 is positive or negative for a target analyte through the operation of the processor 1620.

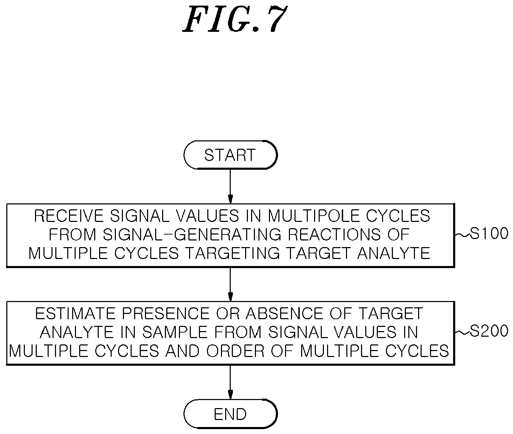

FIG. 7 is a flowchart of a positive/negative determining method according to an embodiment. The positive/negative determining method according to this flowchart can be performed by the positive and negative reading apparatus 1000 described above. In addition, the positive and negative reading method shown in FIG. 7 is merely an example, and the spirit of the present disclosure is not limited to that shown in FIG. 7.

Referring to FIG. 7, the positive/negative determining apparatus 1000 may receive a signal value in each of a plurality of cycles from a plurality of cycle signal-generating reactions targeting a target analyte in a sample for which positive/negative is to be determined through the input unit 1100 (S 100).

This signal value may be received from the detection apparatus 3000 as described above, but is not limited thereto.

In addition, the positive/negative determining apparatus 1000 uses a previously trained model to determine the presence or absence of the target analyte in the sample from signal values in the plurality of cycles and the order of the plurality of cycles through the positive/negative determining unit 1300 (S200). Here, the positive/negative determining unit 1300 can receive the above-described data set from the input unit 1100 or the pre-processor 1200, as described above.

According to another embodiment of the present disclosure, description of the same parts applied to the positive/negative determining apparatus 1000 is omitted as appropriate to avoid duplication.

There may be only one target analyte to be detected in a sample contained in one reaction vessel. A signal dependent on the presence of the target analyte can be detected during a signal generation process using a signal generating composition containing a label.

The positive/negative determining apparatus 1000 also receives this signal from the detection apparatus 3000 and determines whether the signal is positive or negative.

There may be two or more target analytes to be detected in a sample contained in one reaction vessel. In this case, the reaction vessel contains signal generating compositions that provide signals depending on the presence of the target analytes and can provide signals indicating the presence of the target analytes through a signal-generating reaction.

Depending on the signal generation method used, signals with different properties (e.g., signals of different wavelengths) can be generated for respective target analytes, and a distinct data set for each target analyte can be obtained. A positive/negative determining unit for each target analyte may be provided separately, and the corresponding data set can be analyzed in the corresponding positive/negative determining unit to check the presence of the target analyte.

Meanwhile, depending on the signal generation means used, signals with substantially the same properties (for example, signals of the same wavelength) can be generated for a plurality of target analytes. In this case, through a series of processes, a distinct data set for each target analyte can be obtained. A positive/negative determining unit for each target analyte is provided separately, and the corresponding data set can be analyzed in the corresponding positive/negative determining unit to check the presence of the target analyte.

Hereinafter, this will be described in more detail. In an implementation example, the input unit 1100 of the positive/negative determining apparatus 1000 receives a signal detected from one analysis sample vessel (reaction tube) from the detection apparatus. However, the signal is provided by a method of detecting a plurality of target analytes using a single type of label. Description will be made on the assumption that this signal is received.

In one implementation example, the pre-processor 1200 of the positive/negative determining apparatus 1000 performs a pre-processing operation for sorting or separating the above-described signal received by the input unit 1100 from the detection apparatus 3000 as a signal for each of the plurality of target analytes. This sorting or separation operation is described in known methods (e.g., WO2015147370, WO2015147377, WO2015147382, and WO2015147412). The sorting or separation process will be described in more detail with reference to the known method described above.

For example, in the case of detecting a first target analyte and a second target analyte in one reaction vessel, if a first signal generating composition is used for detection of the first target analyte, the first signal generating composition can generate a signal indicating the presence of the first target analyte at a first temperature in a signal-generating reaction. Additionally, a signal indicating the presence of the first target analyte can be generated even at a second temperature. Meanwhile, if a second signal generating composition is used for detection of the second target analyte, the second signal generating composition can prevent a signal from being generated even if the second target analyte is present at the first temperature in the signal-generating reaction. At the same time, the second signal generating composition can generate a signal indicating the presence of the second target analyte at the second temperature. Here, the signal-generating reaction includes a reaction carried out by the first signal generating composition that generates a signal at each of the first temperature and the second temperature when the target analyte is present in the sample. In addition, the signal-generating reaction may further include a reaction carried out by the second signal generating composition that does not generate a signal at the first temperature but generates a signal at the second temperature when a target analyte different from the target analyte is present in the sample.

In one implementation example, the first temperature is a higher than the second temperature. In one implementation example, the first temperature is lower than the second temperature.

In this respect, depending on the signal generating composition, there may be a first temperature range in which a signal for the first target analyte is generated whereas a signal for the second target analyte is not generated. On the other hand, there may be a second temperature range in which signals are generated for both target analytes.

Accordingly, in consideration of the above-described two temperature ranges (first temperature range and second temperature range), detection temperatures for the two target analytes may be determined.

For example, when the first temperature is higher than the second temperature, the relatively high detection temperature among the detection temperatures may be selected from the first temperature range. At a relatively high detection temperature, even if both the first target analyte and the second target analyte are present in one sample, only a signal corresponding to the first target analyte is generated.

In addition, a relatively low detection temperature among the detection temperatures may be selected from the second temperature range. At the relatively low detection temperature, when both the first target analyte and the second target analyte are present in one sample, not only a signal corresponding to the first target analyte but also a signal corresponding to the second target analyte is generated.

That is, one of the two target analytes has a relatively high detection temperature determined by the corresponding signal generating composition (first signal generating composition), and the other has a relative low detection temperature determined by the corresponding other signal generating composition (second signal generating composition).

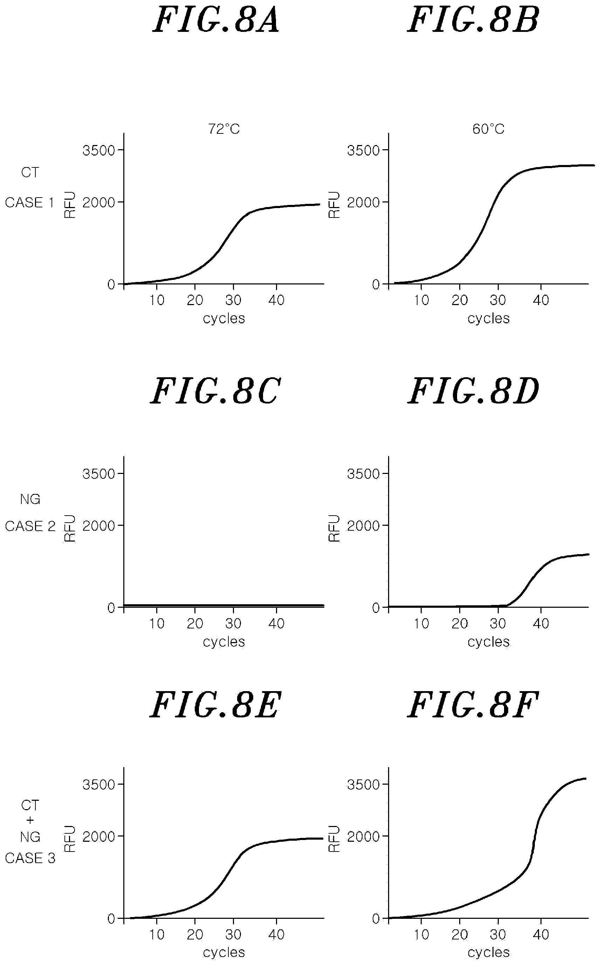

Based on this, the pre-processor 1200 according to one embodiment can separate signals indicating the presence of two target analytes through a predetermined operation, and transmit the separated results to the positive/negative determining unit 1300. The "predetermined operation" will be described with reference to FIG. 8.

FIGS. 8A to 8F show signal value results for cycles obtained by reacting various combinations of CT (Chlamydia Trachomatis) that is the first target substance and NG (Neisseria Gonorrhoeae) that is the second target substance with the first signal generating composition and the second signal generating composition. The first temperature is 72°C and the second temperature is 60°C. Among the substances, CT is an example of a target analyte with a relatively high detection temperature, and NG is an example of a target analyte with the relatively low detection temperature.

As shown in FIGS. 8A and 8E, when CT is present in the sample (CASE 1 and CASE 3), a signal is detected at 72°C. In addition, in the case of FIGS. 8C and 8D in which only NG is present in the sample (CASE 2), a signal is detected in FIG. 8D at 60°C, but no signal is detected in FIG. 8C at 72°C.

In FIGS. 8A, 8C, and 8E, at 72°C, which is the relatively high detection temperature, a signal for CT with the relatively high detection temperature is generated, but for NG with the relatively low detection temperature, no signal is generated. Accordingly, it can be ascertained that at least the presence of CT having the relatively high detection temperature can be determined by detecting a signal at the relatively high detection temperature. That is, when the data set in FIGS. 8A, 8C, and 8E, which is a signal at the relatively high detection temperature, is input to the positive/negative determining unit 1300, whether the CT in the sample is positive or negative can be determined.

Signals detected at the relatively low detection temperature may include signals for both CT and NG. However, by using signals obtained at the relatively high detection temperature, the signal for CT can be removed from the signals obtained at the relatively low detection temperature. Accordingly, a signal indicating the presence or absence of NG, that is, a data set, can be obtained. When the data set is input to the positive/negative determining unit 1300, whether NG in the sample is positive or negative can be determined.

For example, if it is necessary to determine the presence of NG in the sample after CT is previously determined to be present in the sample (FIG. 8A or FIGS. 8E), an operation of subtracting the graph of FIG. 8A from the graph of FIG. 8B is performed, and when the subtracted result is input to the positive/negative determining unit 1300, the presence of NG in the sample can be determined. At this time, the graphs of FIGS. 8A and 8B show a slight difference in the size, that is, amplitude, of the RFU in the same cycle. There are various techniques to correct this difference. However, since these techniques are already known, detailed descriptions thereof will be omitted.

Alternatively, if the presence of NG in the sample needs to be determined after CT has been previously determined to be not present in the sample (FIG. 8C), the presence of NG in the sample can be determined when the graph of FIG. 8B is input to the positive/negative determining unit 1300.

As discussed above, according to one embodiment, whether a sample is positive or negative for a target analyte can be determined by a model trained using a deep learning method. Accordingly, the rapidity and accuracy of positive/negative determination results can be improved.

Meanwhile, the above-described positive/negative determining method may be implemented in the form of a computer program programmed to execute each step included in this method. In this case, the computer program may be included in a computer-readable recording medium. That is, according to various implementation examples of the present disclosure, the positive/negative determining method can be implemented in the form of a computer-readable recording medium storing the above-described computer program or a computer program stored in the above-described computer-readable recording medium.

Combinations of each block of the block diagram and each step of the flowchart attached to the present disclosure may be performed by computer program instructions. Since these computer program instructions can be installed in an encoding processor of a general-purpose computer, special-purpose computer, or other programmable data processing equipment, the instructions executed through the encoding processor of the computer or other programmable data processing equipment generate means for performing functions described in each block of the block diagram and each step of the flowchart. Since these computer program instructions can also be stored in a computer-usable or computer-readable memory that can be directed to a computer or other programmable data processing equipment to implement functions in a specific method, the instructions stored in the computer-usable or computer-readable memory can also produce manufactured items containing instruction means for performing the functions described in each block of the block diagram or each step of the flowchart. Since the computer program instructions can also be installed in a computer or other programmable data processing equipment, a series of operational steps can be performed on the computer or other programmable data processing equipment to create a process that is executed by the computer, thereby providing steps for executing the functions described in each block of the block diagram and each step of the flowchart by the instructions performed through the computer or other programmable data processing equipment.

Additionally, each block or each step may represent a module, segment, or portion of code that includes one or more executable instructions for executing specified logical function(s). Additionally, it should be noted that in some alternative embodiments, it is possible for the functions mentioned in blocks or steps to occur out of order. For example, two blocks or steps shown in succession may in fact be performed substantially simultaneously, or the blocks or steps may sometimes be performed in reverse order depending on the corresponding function.

The above description is merely an illustrative explanation of the technical idea of the present disclosure, and those skilled in the art will be able to make various modifications and variations without departing from the essential quality of the present disclosure. Accordingly, the embodiments disclosed in the present disclosure are not intended to limit the technical idea of the present disclosure, but rather to explain the same, and the scope of the technical idea of the present disclosure is not limited by such embodiments. The scope of protection of the present disclosure should be interpreted in accordance with the claims below, and all technical ideas within the scope equivalent thereto should be construed as being included in the scope of rights of the present disclosure.

## Claims

1. A positive/negative determining apparatus comprising:
an input unit that receives signal values from a signal-generating reaction of a plurality of cycles targeting a target analyte, wherein the signal-generating reaction generates a signal dependent on a presence of the target analyte in a sample; and
a positive/negative determining unit pre-trained to estimate presence/absence of the target analyte in the sample from the signal values in the plurality of cycles and a sequence of the plurality of cycles.

2. The apparatus of claim 1, wherein the target analyte includes a target nucleic acid molecule.

3. The apparatus of claim 1, wherein the signal dependent on the presence of the target analyte includes fluorescence signal.

4. The apparatus of claim 1, wherein the signal-generating reaction includes nucleic acid amplification reaction.

5. The apparatus of claim 1, wherein the positive/negative determining unit obtains the signal value from the input unit sequentially according to the sequence of the cycles.

6. The apparatus of claim 1, wherein the positive/negative determining unit estimates the presence/absence of the target analyte in the sample after obtaining all of the signal values of the plurality of cycles from the input unit.

7. The apparatus of claim 1, wherein in a training process of the positive/negative determining unit, training data including training input data and training correct answer data are used,
the training input data includes signal values for a plurality of training cycles, which are previously obtained by the signal generation reaction for generating a signal dependent on the presence of the target analyte, and information about a sequence of the training cycles, and
the training correct answer data includes information on the presence/ absence of the target analytes.

8. The apparatus of claim 7, wherein in the training process of the positive/negative determining unit, the signal values for the plurality of training cycles among the training input data are sequentially input to the positive/negative determining unit according to the sequence of the cycles.

9. The apparatus of claim 8, wherein the training process of the positive/negative determining unit includes:
comparing a result estimated in response to the training input data input to the positive/negative determining unit and the training correct answer data; and
training the positive/negative determining unit to reduce a difference derived through the comparison.

10. The apparatus of claim 9, wherein in the process of training the positive/negative determining unit to reduce the difference, a backpropagation method is used.

11. The apparatus of claim 7, wherein when the positive/negative determining unit includes a BERT (Bidirectional Encoder Representations from Transformers) model, the positive/negative determining unit includes:
a body part that is trained based at least in part on the training input data; and
a fine-tuning part that is combined with the trained body part, and is trained to infer whether the training input data is positive or negative when the training input data is input to the trained body part.

12. The apparatus of claim 11, wherein the body part is trained according to a semi-supervised learning method.

13. The apparatus of claim 12, wherein the semi-supervised learning comprising:
masking a part of the training input data and then matching the masked part; and
matching an order or a precedence relation of signal values having an order included in the training input data.

14. The apparatus of claim 11, wherein the fine-tuning part is trained according to a supervised-learning method.

15. The apparatus of claim 11, wherein the positive/negative determining unit includes:
an encoder part that generates a context vector from the signal values in the plurality of cycles and the sequence of each cycle; and
a binary classification part that estimates the presence/absence of the target analyte in the sample from the context vector.

16. The apparatus of claim 1, wherein in a training process when the positive/negative determining unit includes a recurrent neural network model or a long short-term memory model,
the positive/negative determining unit is trained such that a weight for a signal value of any one cycle among the plurality of cycles and a weight for a hidden state value calculated for a cycle that temporally precedes the one cycle are updated so that a difference between a result output from an output terminal of the positive/negative determining unit in response to training input data input to an input terminal of the positive/negative determining unit and training correct answer data is reduced.

17. The apparatus of claim 1, wherein an input/output structure of the positive/negative determining unit when the positive/negative determining unit includes a recurrent neural network model or a long short-term memory model is a many-to-one structure that outputs one data when receiving multiple data.

18. The apparatus of claim 1, wherein the positive/negative determining unit is provided in the positive/negative determining apparatus for each predefined factor that affects a signal generation of the signal-generating reaction.

19. The apparatus of claim 18, wherein the predefined factor includes reaction conditions in a nucleic acid amplification reaction.

20. The apparatus of claim 19, wherein the reaction conditions are a reaction medium used for the nucleic acid amplification reaction.

21. The apparatus of claim 20, wherein the reaction medium is one or more substances selected from a group consisting of pH-related substances, ionic strength-related substances, enzymes and enzyme stabilization-related substances.

22. The apparatus of claim 21, wherein the pH-related substances includes buffer, the ionic strength-related substances includes ionic substances, and the enzyme stabilization-related substances includes sugar.

23. The apparatus of claim 1, further comprising:
a pre-processor that performs a predetermined pre-processing operation on the signal value in each of the plurality of cycles,
wherein the positive/negative determining unit estimates the presence/absence of the target analyte in the sample from the signal value on which the pre-processing operation is performed by the pre-processor.

24. The apparatus of claim 23, wherein the pre-processing operation includes a correction operation for an abnormal signal among the signal values in the plurality of cycles, a smoothing operation for the signal values in the plurality of cycles, or a baseline subtraction operation for subtracting a baseline signal value regressed from the signal values in the plurality of cycles from the signal values in the plurality of cycles.

25. A positive/negative determining method executed by a positive/negative determining apparatus, comprising:
receiving signal values from a signal-generating reaction of a plurality of cycles targeting a target analyte, wherein the signal-generating reaction generates a signal dependent on a presence of the target analyte in a sample; and
estimating presence/absence of the target analyte in the sample from the signal values in the plurality of cycles and a sequence of the plurality of cycles by a pre-trained model.

26. A computer-readable storage medium storing a computer program, wherein the computer program includes instructions for causing a processor to perform a positive/negative determining method targeting a target analyte executed by a positive/negative determining apparatus, and
wherein the method includes:
receiving signal values from a signal-generating reaction of a plurality of cycles targeting a target analyte, wherein the signal-generating reaction generates a signal dependent on a presence of the target analyte in a sample; and
estimating presence/absence of the target analyte in the sample from the signal values in the plurality of cycles and a sequence of the a plurality of cycles by a pre-trained model.
